# FASCICULE DE BREVET EUROPEEN

(11) **EP 2 516 473 B1**
(45) Date de publication et mention de la délivrance du brevet: **30.09.2020**
(21) Numéro de dépôt: 10814709.1
(22) Date de dépôt: 23.12.2010
(51) Int. Cl.: C08B 37/02, C08B 37/08, C08B 37/00, C08B 1/00, A61K 47/36

(54) **POLYSACCHARIDES ANIONIQUES FONCTIONNALISES PAR UN DERIVE D'ACIDE HYDROPHOBE**
ANIONISCHE POLYSACCHARIDE FUNKTIONALISIERT MIT DEM DERIVAT EINER HYDROPHOBEN SÄURE
ANIONIC POLYSACCHARIDES FUNCTIONALISED WITH A DERIVATIVE OF HYDROPHOBIC ACID

(30) Priorité: 23.12.2009 WO PCT/IB2009/007899; 07.04.2010 FR 1001439; 07.04.2010 US 282836 P
(43) Date de publication de la demande: 31.10.2012
(73) Titulaire: Adocia, 69003 Lyon (FR)
(72) Inventeur: CHARVET, Richard, F-69140 Rillieux La Pape (FR); SOULA, Rémi, 69330 Meyzieu (FR); SOULA, Olivier, F-69330 Meyzieu (FR)
(74) Mandataire: Tripoz, Inès
(86) Numéro de dépôt international: PCT/IB2010/056051
(87) Numéro de publication internationale: WO 2011/077405

(56) Documents cités:
- EP-A1- 1 493 754
- WO-A1-2007/116143
- WO-A1-2008/038111
- WO-A1-2009/127940

## Description

La présente invention concerne de nouveaux polymères biocompatibles à base de polysaccharides anioniques fonctionnalisés par un dérivé d'acide hydrophobe pouvant être utiles, notamment pour l'administration de principe(s) actif(s) (PA) aux hommes ou aux animaux dans un but thérapeutique et/ou prophylactique.

Les polysaccharides anioniques fonctionnalisés par un dérivé d'acide hydrophobe présentent du fait de leur structure et de leur biocompatibilité un intérêt particulier en pharmacie et plus particulièrement dans le domaine de la stabilisation de principes actifs protéiques par la formation de complexes.

Les acides hydrophobes présentent un intérêt dans la formulation de principes actifs pharmaceutiques, notamment, en raison de leur caractère hydrophobe permettant de moduler l'hydrophobicité des polymères sur lesquels ils peuvent être greffés.

Leur biocompatibilité est excellente dans la mesure où ils jouent un rôle dans de nombreux processus biochimiques et sont présents sous forme estérifiée dans la plupart des tissus.

On connait des techniques de greffage d'acides hydrophobes directement sur les fonctions hydroxyles du dextrane. Ces techniques conduisent à la formation d'un ester d'acide hydrophobe directement sur le squelette du polysaccharide. Cela a été réalisé avec des dérivés activés d'acides gras, tels que des anhydrides (Novak LJ, Tyree JT (1960) US Patent 2954372 ; Mooroka, T. et al., J. Appl. Polym. Sci. 1984, 29, 3981-3990), des chlorures d'acyles (Ehrhardt S et al. (1997) EP792888 ; Aburto, J et al., J. Appl. Polym. Sci. 1999, 74, 1440-1451), des N-acyl urées (Nichifor, Marieta et al., Eur. Polym. J. 1999, 35, 2125-2129) etc.. Des acides hydrophobes ont également été greffés par transestérification à partir de leurs dérivés esters (Laletin, AJ et al., Vysokomol Soedin Ser A 1968, 10, 652) et par des techniques d'activation avec le chlorure de tosyle par exemple (Heinze, T. et al., Cellulose 2003, 10, 283-296). Ces méthodes n'ont été mises en œuvre qu'avec des polysaccharides neutres puisqu'elles ne sont pas compatibles avec la présence de fonctions carboxylates sur le polysaccharide.

EP 1 493 754 décrit des polysaccharides contenant des groupements phosphorylcholine, destinés à l'administration de principes actifs.

La présente invention concerne de nouveaux polysaccharides anioniques fonctionnalisés par au moins un dérivé d'acide hydrophobe tels que décrits à la revendication 1. Ces nouveaux polysaccharides anioniques comportant des groupes hydrophobes ont une bonne biocompatibilité et leur hydrophobicité est facilement modulable sans altérer la biocompatibilité ou la stabilité.

Elle concerne également une méthode de synthèse qui fait l'objet de la revendication 7 permettant de résoudre les problèmes de synthèse supra cités. Cette méthode a permis d'obtenir lesdits polysaccharides anioniques fonctionnalisés par des acides hydrophobes.

On entend par anionique un polysaccharide qui comprend des fonctions carboxyles non fonctionnalisées et salifiables.

On entend par monofonctionnalisé un polysaccharide anionique qui est fonctionnalisé par un groupement hydrophobe unique et qui ne comporte pas d'autres substituants.

On entend par degré de fonctionnalisation le nombre de fonctions M-Ach par unité saccharidique ou en d'autres termes le nombre total de fonctions M-Ach rapporté au nombre total d'unités saccharidiques. Cette notion peut également être exprimée en fraction molaire des fonctions hydroxyles du polysaccharide fonctionnalisées par M-Ach.

On entend par degré de conversion le nombre de fonctions hydroxyles converties en carboxylates par unité saccharidique ou en d'autres termes le nombre total de fonctions hydroxyles converties en carboxylates rapporté au nombre total d'unités saccharidiques. Cette notion peut être également exprimée en fraction molaire. Par exemple des polysaccharides pour lesquels le degré de conversion des fonctions hydroxyles en carboxylates par unité saccharidique est égal ou supérieur à 0,15 sont des polysaccharides pour lesquels au moins 15 fonctions carboxyles pour 100 unités saccharidiques ont été greffées.

On entend par degré de polymérisation m le nombre moyen d'unités répétitives (monomères) par chaine de polymère. Il se calcule en divisant la masse molaire moyenne en nombre par la masse moyenne du motif répété.

On entend par masse molaire moyenne en nombre (Mₙ) la moyenne arithmétique des masses de chacune des chaînes de polymère. Ainsi pour un nombre nᵢ de chaînes i de masse molaire Mᵢ, on a Mₙ = (ΣᵢnᵢMᵢ)/(Σᵢnᵢ).

La masse molaire moyenne en poids (M_{w}) est obtenue par M_{w} = (ΣᵢnᵢMᵢ²)/(ΣᵢnᵢMᵢ), nᵢ étant le nombre de chaines de polymère i de masse molaire Mᵢ,

Les polymères peuvent également être caractérisés par la distribution de longueurs de chaînes, aussi appelée indice de polydispersité (Ip), et est égal au M_{w} divisé par le Mₙ.

Ledit polysaccharide anionique monofonctionnalisé par un dérivé d'acide hydrophobe est choisi parmi les polysaccharides comportant des fonctions carboxyles de formule générale V : 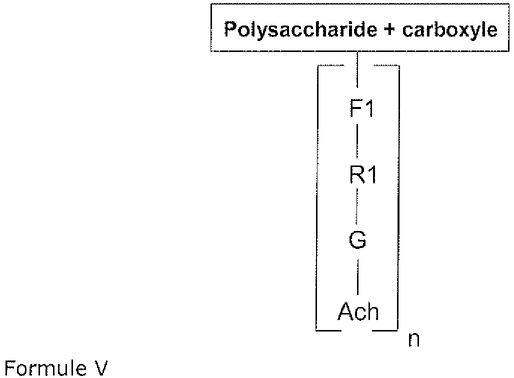
- dans laquelle, n représente le degré de fonctionnalisation des fonctions carboxyles du polysaccharide par un enchaînement F₁-R₁-G-Ach et est compris entre 0,01 et 0,7,
   ▪ F₁ étant soit une fonction amide, soit une fonction ester, soit une fonction thioester,
   ▪ G étant soit une fonction ester, soit une fonction amide, soit une fonction thioester,
   ▪ R₁ étant un radical divalent constitué d'une chaîne comprenant entre 1 et 15 carbones, éventuellement branchée et/ou insaturée, éventuellement comprenant un ou plusieurs hétéroatomes, tels que 0, N ou/et S, éventuellement comprenant un ou plusieurs cycles ou hétérocycles saturés, insaturés ou aromatiques et résultant de la réaction d'un précurseur R₁' ayant au moins deux fonctions réactives, identiques ou différentes choisies dans le groupe constitué par les fonctions alcool, amine et thiol.
   ▪ Ach étant un radical, produit du couplage entre la fonction carboxyle de l'acide hydrophobe et au moins une fonction réactive portée par le précurseur R₁' du radical divalent R₁,
   et lorsque la fonction carboxyle du polysaccharide n'est pas fonctionnalisée par F₁-R₁-G-Ach, alors le ou les fonctions carboxyles du polysaccharide sont des carboxylates de cation, alcalin de préférence comme Na⁺ ou K⁺.

Dans un mode de réalisation, F₁ est une fonction amide, G est une fonction ester, R₁' est une alcoolamine et Ach est issu d'un acide hydrophobe.

Dans un mode de réalisation, F₁ est une fonction amide, G est une fonction thioester, R₁' est un aminothioalcool et Ach est issu d'un acide hydrophobe.

Dans un mode de réalisation, F₁ est une fonction amide, G est une fonction amide, R₁' est une diamine et Ach est issu d'un acide hydrophobe.

Dans un mode de réalisation, F₁ est une fonction ester, G est une fonction amide, R₁' est un alcoolamine et Ach est issu d'un acide hydrophobe.

Dans un mode de réalisation, F₁ est une fonction ester, G est une fonction ester, R₁' est un dialcool et Ach est issu d'un acide hydrophobe.

Dans un mode de réalisation, F₁ est une fonction ester, G est une fonction thioester, R₁' est un hydroxythioalcool et Ach est issu d'un acide hydrophobe.

Dans un mode de réalisation, F₁ est une fonction thioester, G est une fonction amide, R₁' est un aminothioalcool et Ach est issu d'un acide hydrophobe.

Dans un mode de réalisation, F₁ est une fonction thioester, G est une fonction ester, R₁' est un hydroxythioalcool et Ach est issu d'un acide hydrophobe.

Dans un mode de réalisation, F₁ est une fonction thioester, G est une fonction thioester, R₁' est un dithioalcool et Ach est issu d'un acide hydrophobe.

Dans un mode de réalisation, le précurseur du groupement R₁, R₁' est caractérisé en ce qu'il est choisi parmi les dialcools.

Dans un mode de réalisation, les dialcools sont choisis dans le groupe constitué par le glycérol, le diglycérol et le triglycérol.

Dans un mode de réalisation, le dialcool est la triéthanofamine.

Dans un mode de réalisation, les dialcools sont choisis dans le groupe constitué par le diéthylèneglycol et le triéthylèneglycol.

Dans un mode de réalisation, les dialcools sont choisis dans le groupe constitué par les polyéthylèneglycols.

Dans un mode de réalisation, les précurseurs des groupements R₁ et R₁' sont caractérisés en ce qu'ils sont choisis parmi les diamines.

Dans un mode de réalisation, les diamines sont choisies dans le groupe constitué par l'éthylène diamine et la lysine et ses dérivés.

Dans un mode de réalisation, les diamines sont choisies dans le groupe constitué par la diéthylèneglycoldiamine et la triéthylèneglycoldiamine.

Dans un mode de réalisation, les précurseurs des groupements R₁ et R₁' sont caractérisés en ce qu'ils sont choisis parmi les alcoolamines.

Dans un mode de réalisation, les alcoolamines sont choisies dans le groupe constitué par l'éthanolamine, l'amino-2-propanol, l'isopropanolamine, le 3-amino-1,2-propanediol, la diéthanolamine, la diisopropanolamine, la trométhamine (Tris) et le 2-(2-aminoéthoxy)éthanol.

Dans un mode de réalisation, les alcoolamines sont choisies dans le groupe constitué par les acides aminés réduits.

Dans un mode de réalisation les acides aminés réduits sont choisis dans le groupe constitué par l'alaninol, le valinol, le leucinol, l'isoleucinol, le prolinol et le phénylalaninol.

Dans un mode de réalisation, les alcoolamines sont choisies dans le groupe constitué par les acides aminés chargés.

Dans un mode de réalisation, les acides aminés chargés sont choisis dans le groupe constitué par la sérine et la thréonine.

Dans un mode de réalisation, l'acide hydrophobe est choisi parmi les acides gras.

Dans un mode de réalisation, les acides gras sont choisis dans le groupe constitué par les acides constitués d'une chaîne alkyle insaturée ou saturée, ramifiée ou non ramifiée, comprenant de 6 à 50 carbones.

Dans un mode de réalisation, les acides gras sont choisis dans le groupe constitué par les acides gras linéaires.

Dans un mode de réalisation, les acides gras linéaires sont choisis dans le groupe constitué par l'acide caproïque, l'acide oenanthique, l'acide caprylique, l'acide caprique, l'acide nonanoïque, l'acide décanoïque, l'acide undécanoïque, l'acide dodécanoïque, l'acide palmitique, l'acide stéarique, l'acide arachidique, l'acide béhénique, l'acide tricosanoïque, l'acide lignocérique, l'acide heptacosanoïque, l'acide octacosanoïque et l'acide mélissique.

Dans un mode de réalisation, les acides gras sont choisis dans le groupe constitué par les acides gras insaturés,

Dans un mode de réalisation, les acides gras insaturés sont choisis dans le groupe constitué par l'acide myristoléique, l'acide palmitoléique, l'acide oléique, l'acide élaidique, l'acide linoléique, l'acide alpha-linoléique, l'acide arachidonique, l'acide eicosapentaenoïque, l'acide erucique et l'acide docosahexaenoïque.

Dans un mode de réalisation, les acides gras sont choisis dans le groupe constitué par les acides de la bile et leurs dérivés.

Dans un mode de réalisation, les acides de la bile et leurs dérivés sont choisis dans le groupe constitué par l'acide cholique, l'acide déhydrocholique, l'acide désoxycholique et l'acide chénodésoxycholique.

Le polysaccharide peut avoir un degré de polymérisation m compris entre 5 et 10000.

Dans un mode de réalisation, il a un degré de polymérisation m compris entre 10 et 1000.

Dans un autre mode de réalisation, il a un degré de polymérisation m compris entre 10 et 500.

L'invention concerne également la synthèse des polysaccharides selon l'invention.

Ladite synthèse comprend une étape d'obtention d'un intermédiaire aminé Ach-G-R₁-NH₂ ou d'un sel d'ammonium Ach-G-R₁-NH₃⁺ dont le contre-ion est un anion choisi parmi les halogénures, les sulfates, les sulfonates, les carboxylates, et une étape de greffage de cet intermédiaire aminé sur une fonction carboxyle d'un polysaccharide, R₁, G et Ach répondant aux définitions données ci-dessus.

Dans un mode de réalisation préféré, l'intermédiaire aminé de formule Ach-G-R₁-NH₂ ou Ach-G-R₁-NH₃⁺ est obtenu par réaction d'un composé de formule G'-R₁-NH₂, G' étant une fonction amine, thiol ou alcool avec la fonction acide de l'acide hydrophobe, R₁, G et Ach répondant aux définitions données ci-dessus.

Si nécessaire dans cette étape d'obtention de l'intermédiaire aminé, les techniques de protection, déprotection bien connues de l'homme de l'art en synthèse peptidique sont utilisées.

De préférence, l'étape de greffage de l'intermédiaire aminé sur une fonction acide du polysaccharide est réalisée en milieu organique.

Si nécessaire dans cette étape d'obtention de l'intermédiaire aminé, les techniques de protection, déprotection bien connues de l'homme de l'art en synthèse peptidique sont utilisées.

De préférence, l'étape de greffage de l'intermédiaire aminé sur une fonction hydroxyle du polysaccharide est réalisée en milieu organique.

Dans un mode de réalisation l'invention concerne un polysaccharide choisi dans le groupe constitué par les polysaccharides suivants :
- dextraneméthylcarboxylate de sodium modifié par l'ester caprylate d'éthanolamine
- dextraneméthylcarboxylate de sodium modifié par l'ester laurate d'éthanolamine (dextrane 40 kDa)
- dextraneméthylcarboxylate de sodium modifié par l'ester laurate d'éthanolamine (dextrane 10 kDa)
- dextraneméthylcarboxylate de sodium modifié par l'ester caprylate de phénylalaninol
- dextraneméthylcarboxylate de sodium modifié par le N-(2-aminoethyl)octanamide
- dextraneméthylcarboxylate de sodium modifié par le N-(2-aminoéthyl)décanamide
- dextraneméthylcarboxylate de sodium modifié par le N-(2-aminoéthyl)dodécanamide (dextrane 10 kDa)
- dextraneméthylcarboxylate de sodium modifié par le N-(2-aminoethyl)dodécanamide (dextrane 40 kDa)
- dextraneméthylcarboxylate de sodium modifié par le 2-(2-[dodécanoylamino]éthoxy)éthylamine
- dextraneméthylcarboxylate de sodium modifié par le 2-(2-{2-[dodécanoylamino]éthoxy}éthoxy)éthylamine
- dextraneméthylcarboxylate de sodium modifié par le 2-(2-{2-[hexadécanoylamino]éthoxy}éthoxy)éthylamine
- dextranmethylcarboxylate de sodium modifié par le 2-(2-amino-éthoxy)éthyl octanoate
- dextraneméthylcarboxylate de sodium modifié par le 2-(2-amino-éthoxy)éthyl dodécanoate
- dextraneméthylcarboxylate de sodium modifié par le 2-(2-amino-éthoxy)éthyl hexadécanoate
- dextraneméthylcarboxylate de sodium modifié par la 2-[(2-octanoylamino-3-phényl)propanoylamino]éthanamine
- dextraneméthylcarboxylate de sodium modifié par la 2-[(2-octanoylamino-3-phenyl)propanoylamino]éthanamine
- N-méthylcarboxylate de sodium dextrane carbamate modifié par le N-(2-aminoethyl)dodécanamide
- dextranesuccinate de sodium modifié par le N-(2-aminoéthyl)dodécanamide
- dextrane modifié par le N-méthylcarboxylate de sodium carbamate et le N-(2-aminoéthyl)dodécanamide carbamate

L'invention concerne également une composition pharmaceutique comprenant l'un des polysaccharides selon l'invention tel que décrit précédemment et au moins un principe actif comme décrite à la revendication 8.

Le principe actif peut être choisi dans le groupe constitué par les protéines, les glycoprotéines, les peptides et les molécules thérapeutiques nonpeptidiques.

On entend par principe actif un produit sous forme d'entité chimique unique ou sous forme d'une combinaison ayant une activité physiologique. Ledit principe actif peut être exogène c'est à dire qu'il est apporté par la composition selon l'invention. Il peut également être endogène, par exemple les facteurs de croissance qui vont être sécrétés dans une plaie pendant la première phase de cicatrisation et qui pourront être retenus sur ladite plaie par la composition selon l'invention.

Selon les pathologies visées elle est destinée à un traitement local ou systémique.

Dans le cas des libérations locale et systémique, les modes d'administration envisagés sont par voie intraveineuse, sous-cutanée, intradermique, transdermique, intramusculaire, orale, nasale, vaginale, oculaire, buccale, pulmonaire etc.

Les compositions pharmaceutiques selon l'invention sont soit sous forme liquide, en solution aqueuse, soit sous forme de poudre, d'implant ou de film. Elles comportent en outre les excipients pharmaceutiques classiques bien connus de l'homme de l'art.

En fonction des pathologies et des modes d'administration les compositions pharmaceutiques pourront avantageusement comporter, en outre, des excipients permettant de les formuler sous forme de gel, d'éponge, de solution injectable, de solution buvable, de lyoc etc.

L'invention concerne également une composition pharmaceutique, caractérisée en ce qu'elle est administrable sous forme de stent, de film ou « coating » de biomatériaux implantables, d'implant.

### Exemple 1 : dextraneméthylcarboxylate de sodium modifié par l'ester caprylate d'éthanolamine

### Polymère 1

8 g (soit 148 mmol de fonctions hydroxyles) de dextrane de masse molaire moyenne en poids d'environ 40 kg/mol (Pharmacosmos) sont solubilisés dans de l'eau à 42 g/L. A cette solution sont ajoutés 15 mL de NaOH 10 N (148 mmol NaOH). Le mélange est porté à 35°C puis 23 g (198 mmol) de chloroacétate de sodium sont ajoutés. La température du milieu réactionnel est portée à 60°C à 0,5°C/min puis maintenue à 60°C pendant 100 minutes. Le milieu réactionnel est dilué avec 200 mL d'eau, neutralisé à l'acide acétique et purifié par ultrafiltration sur membrane PES de 5 kD contre 6 volumes d'eau. La solution finale est dosée par extrait sec pour déterminer la concentration en polymère ; puis dosée par dosage acide/base dans de l'eau/acétone 50 / 50 (V/V) pour déterminer le degré de conversion des fonctions hydroxyles en méthylcarboxylates,

D'après l'extrait sec : [polymère] = 31,5 mg/g
D'après le dosage acide/base : le degré de conversion des fonctions hydroxyles en fonctions méthylcarboxylates est de 1,06 par motif saccharidique.

La solution de dextraneméthylcarboxylate de sodium est passée sur une résine Purolite (anionique) pour obtenir le dextraneméthylcarboxylique acide qui est ensuite lyophilisé pendant 18 heures.

L'ester caprylate d'éthanolamine, sel d'acide paratoluènesulfonique est obtenu selon le procédé décrit dans le brevet (Kenji, M et al., US4826818).

7,5 g de dextraneméthylcarboxylique acide (35,57 mmol fonction méthylcarboxylique acide) sont solubilisés dans le DMF à 60 g/L puis refroidis à 0°C. 2,41 g d'ester caprylate d'éthanolamine, sel d'acide paratoluènesulfonique (6,71 mmol) sont mis en suspension dans du DMF à 100 g/L. 0,68 g de triéthylamine (6,71 mmol) est ensuite ajouté à cette suspension. Une fois la solution de polymère à 0°C, une solution de NMM (3,96 g, 39,1 mmol) dans le DMF (530 g/L) et 4,25 g (39,1 mmol) de EtOCOCl sont ensuite ajoutés. Après 10 minutes de réaction, la suspension d'ester caprylate d'éthanolamine est ajoutée. Le milieu est ensuite maintenu à 10°C pendant 45 minutes. Le milieu est ensuite chauffé à 50°C. Une solution d'imidazole (7,9 g dans 13 mL d'eau) et 39 mL d'eau sont ajoutés dans le milieu réactionnel. La solution de polymère est ultrafiltrée sur membrane PES 10 kD contre 6 volumes de solution NACl 0,9 %, 3 volumes de soude 0,01N, 7 volumes de solution NaCl 0,9 % puis 3 volumes d'eau. La concentration de la solution de polymère est déterminée par extrait sec. Une fraction de solution est lyophilisée et analysée par RMN ¹H dans D₂O pour déterminer le taux de fonctions acides converties en amide d'ester caprylate d'éthanolamine.

D'après l'extrait sec : [Polymère 1] = 29,1 mg/g
D'après la RMN ¹H : le degré de fonctionnalisation des acides par l'ester caprylate d'éthanolamine par unité saccharidique est de 0,15.

### Exemple 2: dextraneméthylcarboxylate de sodium modifié par l'ester laurate d'éthanolamine(dextrane 40 kDa)

### Polymère 2

L'ester laurate d'éthanolamine, sel d'acide paratoluènesulfonique est obtenu selon le procédé décrit dans le brevet (Kenji, M et al., US4826818).

Par un procédé similaire à celui décrit à l'Exemple 1, un dextraneméthylcarboxylate de sodium, modifié par l'ester laurate d'éthanolamine est obtenu.

D'après l'extrait sec : [Polymère 2] = 21,2 mg/g
D'après la RMN ¹H : le degré de fonctionnalisation des acides par l'ester laurate d'éthanolamine par unité saccharidique est de 0,09.

### Exemple 3 : dextraneméthylcarboxylate de sodium modifié par l'ester laurate d'éthanolamine (dextrane 10 kDa)

### Polymère 3

Par un procédé similaire à celui décrit à l'Exemple 2, un dextraneméthylcarboxylate de sodium, synthétisé selon le procédé décrit dans l'Exemple 1 en utilisant un dextrane de masse moléculaire moyenne en poids d'environ 10 kg/mol (Pharmacosmos), modifié par l'ester laurate d'éthanolamine est obtenu.

D'après l'extrait sec : [Polymère 3] = 31,4 mg/g
D'après la RMN ¹H : le degré de fonctionnalisation des acides par l'ester laurate d'éthanolamine par unité saccharidique est de 0,09.

### Exemple 4 : dextraneméthylcarboxylate de sodium modifié par l'ester caprylate de phénylalaninol

### Polymère 4

L'ester caprylate de phénylalaninol, sel d'acide paratoluènesulfonique est obtenu selon le procédé décrit dans le brevet (Kenji, M et al., US4826818).

Par un procédé similaire à celui décrit à l'Exemple 1, un dextraneméthylcarboxylate de sodium modifié par l'ester caprylate de phénylalaninol est obtenu.

D'après l'extrait sec : [Polymère 4] = 25 mg/g
D'après la RMN ¹H : le degré de fonctionnalisation des acides par l'ester caprylate de phénylalaninol par unité saccharidique est de 0,045.

### Exemple 5 : dextraneméthylcarboxylate de sodium modifié par le N-(2-aminoethyl)octanamide

### Polymère 5

Le N-(2-aminoethyl)octanamide est obtenu selon le procédé décrit dans le brevet (Weiner, N. et al., US2387201) à partir du méthyl ester de l'acide octanoïque (Sigma) et de l'éthylènediamine (Roth).

Par un procédé similaire à celui décrit dans l'exemple 1, un lyophilisat de dextraneméthylcarboxylique acide est obtenu à partir d'un dextrane de masse molaire moyenne en poids d'environ 10 kg/mol (Pharmacosmos).

8 g de dextraneméthylcarboxylique acide (37 mmol fonctions méthylcarboxylique acide) sont solubilisés dans le DMF à 70 g/L puis refroidis à 0°C, Une fois la solution de polymère à 0°C, 4,13 g (41 mmol) de NMM et 4,44 g (41 mmol) de EtOCOCl sont ensuite ajoutés. Après 10 min de réaction, 1,34 g de N-(2-aminoethyl)octanamide (9,0 mmol) est introduit et le milieu porté à 30°C durant 90 minutes. Une solution aqueuse d'imidazole à 600 g/L et 40 mL d'eau sont ajoutés et le milieu est ensuite chauffé à 50°C. La solution obtenue est ultrafiltrée sur membrane PES 10 kD contre 6 volumes de solution NaCl 0,9%, 3 volumes de soude 0,01N, 9 volumes de solution NaCl 0,9% puis 3 volumes d'eau. La concentration de la solution de polymère est déterminée par extrait sec. Une fraction de solution est lyophilisée et analysée par RMN ¹H dans D₂O pour déterminer le taux de fonctions acides converties en amide de N-(2-aminoéthyl)octanamide.

D'après l'extrait sec : [Polymére 5] = 24,8 mg/g
D'après la RMN ¹H : le degré de fonctionnalisation des acides par le N-(2-aminoéthyl)octanamide par unité saccharidique est de 0,20.

### Exemple 6 : dextraneméthylcarboxylate de sodium modifié par le N-(2-aminoéthyl)décanamide

### Polymère 6

Le N-(2-aminoéthyl)décanamide est obtenu selon le procédé décrit dans le brevet (Weiner, N. et al., US2387201) à partir du méthyl ester de l'acide décanoïque (Sigma) et de l'éthylènediamine (Roth).

Par un procédé similaire à celui décrit à l'Exemple 5, un dextraneméthylcarboxylate de sodium de masse molaire moyenne en poids de 10 kDa modifié par le N-(2-aminoéthyl)décanamide est obtenu.

D'après l'extrait sec : [Polymère 6] = 23,0 mg/g
D'après la RMN ¹H : le degré de fonctionnalisation des acides par le N-(2-aminoéthyl)décanamide par unité saccharidique est de 0,09.

### Exemple 7 : dextraneméthylcarboxylate de sodium modifié par le N-(2-aminoéthyl)dodécanamide (dextrane 10 kDa)

### Polymère 7

Le N-(2-aminoéthyl)dodécanamide est obtenu selon le procédé décrit dans le brevet (Weiner, N. et al., US2387201) à partir du méthyl ester de l'acide dodécanoïque (Sigma) et de l'éthylènediamine (Roth).

Par un procédé similaire à celui décrit à l'Exemple 5, un dextraneméthylcarboxylate de sodium de masse molaire moyenne en poids de 10 kDa modifié par le N-(2-aminoethyl)dodecanamide est obtenu.

D'après l'extrait sec : [Polymère 7] ≈ 23,8 mg/g.
D'après la RMN ¹H : le degré de fonctionnalisation des acides par le N-(2-aminoéthyl)décanamide par unité saccharidique est de 0,10.

### Exemple 8: dextraneméthylcarboxylate de sodium modifié par le N-(2-aminoethyl)dodécanamide (dextrane 40 kDa)

### Polymère 8

Par un procédé similaire à celui décrit à l'Exemple 7, un dextraneméthylcarboxylate de sodium de masse molaire moyenne en poids de 40 kDa modifié par le N-(2-aminoéthyl)dodécanamide est obtenu.

D'après l'extrait sec : [Polymère 8] = 24,6 mg/g.
D'après la RMN ¹H : le degré de fonctionnatisation des acides par le N-(2-aminoéthyl)décanamide par unité saccharidique est de 0,10.

### Exemple 9 : dextraneméthylcarboxylate de sodium modifié par le 2-(2-[dodécanoylamino]éthoxy)éthylamine

### Polymère 9

Le 2-(2-[dodécanoylamino]éthoxy)éthylamine est obtenu selon le procédé décrit dans le brevet (Weiner, N. et al., US2387201) à partir du méthyl ester de l'acide dodécanoïque (Sigma) et du diéthylèneglycol diamine (Huntsman).

Par un procédé similaire à celui décrit à l'Exemple 5, un dextraneméthylcarboxylate de sodium de masse molaire moyenne en poids de 10 kDa modifié par le 2-(2-[dodécanoylamino]éthoxy)éthylamine est obtenu.

D'après l'extrait sec : [Polymère 9] = 21,4 mg/g
D'après la RMN ¹H : le degré de fonctionnalisation des acides par le 2-(2-[dodécanoylamino]éthoxy)éthylamine par unité saccharidique est de 0,09.

### Exemple 10 : dextraneméthylcarboxylate de sodium modifié par le 2-(2-{2-[dodécanoylamino]éthoxy}éthoxy)éthylamine

### Polymère 10

Le 2-(2-{2-[dodécanoyiamino]éthoxy}éthoxy)éthylamine est obtenu selon le procédé décrit dans le brevet (Weiner, N. et al., US2387201) à partir du méthyl ester de l'acide dodécanoïque (Sigma) et du triéthylèneglycol diamine (Huntsman).

Par un procédé similaire à celui décrit à l'Exemple 5, un dextraneméthylcarboxylate de sodium de masse molaire moyenne en poids de 10 kDa modifié par le 2-(2-{2-[dodécanoylamino]éthoxy}éthoxy)éthylamine est obtenu.

D'après l'extrait sec : [Polymère 10] = 24,9 mg/g
D'après la RMN ¹H : le degré de fonctionnalisation des acides par le 2-(2-{2-[dodécanoylamino]éthoxy}éthoxy)éthylamine par unité saccharidique est de 0,09.

### Exemple 11 : dextraneméthylcarboxylate de sodium modifié par le 2-(2-{2-[hexadécanoylamino]éthoxy}éthoxy)éthylamine

### Polymère 11

Le 2-(2-{2-[hexadécanoylamino]éthoxy}éthoxy)éthylamine est obtenu selon le procédé décrit dans le brevet (Weiner, N. et al., US2387201) à partir du méthyl ester de l'acide palmitique (Sigma) et du triéthylèneglycol diamine (Huntsman).

Par un procédé similaire à celui décrit à l'Exemple 5, un dextraneméthylcarboxylate de sodium de masse molaire moyenne en poids de 10 kDa modifié par le 2-(2-{2-[hexadécanoylamino]éthoxy}éthoxy)éthylamine est obtenu.

D'après l'extrait sec : [Polymère 11] = 22,2 mg/g
D'après la RMN ¹H : le degré de fonctionnalisation des acides par le 2-(2-{2-[hexadécanoylamino]éthoxy}éthoxy)éthylamine par unité saccharidique est de 0,04.

### Exemple 12 : dextranmethylcarboxylate de sodium modifié par le 2-(2-amino-éthoxy)éthyl octanoate

### Polymère 12

Le 2-(2-amino-ethoxy)ethyl octanoate, sel d'acide paratoluènesulfonique est obtenu selon le procédé décrit dans le brevet (Kenji, M et al., US4826818) à partir de l'acide octanoïque (Sigma) et du diéthylèneglycol amine (Sigma).

Par un procédé similaire à celui décrit à l'Exemple 1, un dextraneméthylcarboxylate de sodium de masse molaire moyenne en poids de 10 kDa modifié par le 2-(2-amino-éthoxy)éthyl octanoate est obtenu.

D'après l'extrait sec : [Polymère 12] = 20,3 mg/g
D'après la RMN ¹H : le degré de fonctionnalisation des acides par le 2-(2-amino-éthoxy)éthyl octanoate par unité saccharidique est de 0,19.

### Exemple 13 : dextraneméthylcarboxylate de sodium modifié par le 2-(2-amino-éthoxy)éthyl dodécanoate

### Polymère 13

Le 2-(2-amino-éthoxy)éthyl dodécanoate, sel d'acide paratoluènesulfonique est obtenu selon le procédé décrit dans le brevet (Kenji, M et al., US4826818) à partir de l'acide dodécanoique (Sigma) et du diéthylèneglycol amine (Sigma).

Par un procédé similaire à celui décrit à l'Exemple 1, un dextraneméthylcarboxylate de sodium de masse molaire moyenne en poids de 10 kDa modifié par le 2-(2-amino-éthoxy)éthyl dodécanoate est obtenu.

D'après l'extrait sec : [Polymère 13] = 25,6 mg/g
D'après la RMN ¹H : le degré de fonctionnalisation des acides par le 2-(2-amino-éthoxy)éthyl dodécanoate par unité saccharidique est de 0,10.

### Exemple 14 : dextraneméthylcarboxylate de sodium modifié par le 2-(2-amino-éthoxy)éthyl hexadécanoate

### Polymère 14

Le 2-(2-amino-éthoxy)éthyl hexadécanoate, sel d'acide paratoluènesulfonique est obtenu selon le procédé décrit dans le brevet (Kenji, M et al., US4826818) à partir de l'acide hexadécanoique (Sigma) et du diéthylèneglycol amine (Sigma).

Par un procédé similaire à celui décrit à l'Exemple 1, un dextraneméthylcarboxylate de sodium de masse molaire moyenne en poids de 10 kDa modifié par le 2-(2-amino-éthoxy)éthyl hexadécanoate est obtenu.

D'après l'extrait sec : [Polymère 14] = 19,6 mg/g
D'après la RMN ¹H : le degré de fonctionnalisation des acides par le 2-(2-amino-éthoxy)éthyl hexadécanoate par unité saccharidique est de 0,05.

### Exemple 15 : dextraneméthylcarboxylate de sodium modifié par la 2-[(2-octanoylamino-3-phényl)propanoylamino]éthanamine

### Polymère 15

La N-octanoyl-phénylalanine est obtenue selon le procédé décrit dans la publication (Pal, A et al., Tetrahedron 2007, 63, 7334-7348) à partir de l'éthyle ester de la L-phénylalanine, sel d'acide chlorhydrique (Bachem) et de l'acide caprylique (Sigma).

La 2-[(2-octanoylamino-3-phényl)propanoylamino]éthanamine, sel d'acide chlorydrique, est obtenue selon les procédés décrits dans les publications (Paul, R et al., J. Org. Chem. 1962, 27, 2094-2099 et Dale, D.J. et al., Org. Process. Res. Dev. 2002, 6, 767-772) à partir de la N-octanoyl-phénylalanine et de l'éthylènediamine (Roth).

Par un procédé similaire à celui décrit à l'Exemple 5, un dextraneméthylcarboxylate de sodium de masse molaire moyenne en poids de 10 kDa modifié par la 2-[(2-octanoylamino-3-phényl)propanoylamino]éthanamine est obtenu.

D'après l'extrait sec : [Polymère 15] = 19,9 mg/g
D'après la RMN ¹H : le degré de fonctionnalisation des acides par la 2-[(2-octanoylamino-3-phényl)propanoylamino]éthanamine par unité saccharidique est de 0,13.

### Exemple 16 : dextraneméthylcarboxylate de sodium modifié par la 2-[(2-octanoylamino-3-phenyl)propanoylamino]éthanamine

### Polymère 16

La N-octanoyl-phénylalanine est obtenue selon le procédé décrit dans la publication (Pal, A et al., Tetrahedron 2007, 63, 7334-7348) à partir de l'éthyle ester de la L-phénylalanine, sel d'acide chlorhydrique (Bachem) et de l'acide caprylique (Sigma).

La 2-[(2-octanoylamino-3-phényl)propanoylamino]éthanamine, sel d'acide chlorydrique, est obtenue selon les procédés décrits dans les publications (Paul, R et al., J. Org. Chem. 1962, 27, 2094-2099 et Dale, D.J. et al., Org. Process. Res. Dev. 2002, 6, 767-772) à partir de la N-octanoyl-phényialanine et de l'éthylènediamine (Roth).

Par un procédé similaire à celui décrit à l'Exemple 5, un dextraneméthylcarboxylate de sodium de masse molaire moyenne en poids de 40 kDa modifié par la 2-[(2-octanoylamino-3-phényl)propanoylamino]éthanamine est obtenu.

D'après l'extrait sec : [Polymère 16] = 19,1 mg/g
D'après la RMN ¹H : le degré de fonctionnalisation des acides par la 2-[(2-octanoylamino-3-phényl)proparloylamino]éthanamine par unité saccharidique est de 0,15.

### Exemple 17: N-méthylcarboxylate de sodium dextrane carbamate modifié par le N-(2-aminoethyl)dodécanamide

### Polymère 17

Le N-(2-aminoethyl)dodécanamide est obtenu selon le procédé décrit dans le brevet (Weiner, N. et al., US2387201) à partir du méthyl ester de l'acide dodécanoïque (Sigma) et de l'éthylènediamine (Roth).

11,5 g (soit 0,21 mol de fonctions hydroxyles) de dextrane de masse molaire moyenne en poids d'environ 10 kg/mol (Bachem) sont solubilisés dans un mélange DMF/DMSO. Le mélange est porté à 130°C sous agitation et 13,75 g (0,11 mol) d'éthyle isocyanatoacétate sont progressivement introduits. Après 1h de réaction, le milieu est dilué en eau et purifié par diafiltration sur membrane PES de 5 kD contre NaOH 0,1N, NaCl 0,9% et de l'eau. La solution finale est dosée par extrait sec pour déterminer la concentration en polymère ; puis dosée par dosage acide/base dans de l'eau/acétone 50 / 50 (V/V) pour déterminer le degré de conversion des fonctions hydroxyles en carboxylates.

D'après l'extrait sec : [polymère] = 38,9 mg/g
D'après le dosage acide/base : le degré de conversion des fonctions hydroxyles en fonctions N-méthylcarboxylates carbamates est de 1,08 par motif saccharidique.

La solution de N-méthylcarboxylate de sodium dextrane carbamate est passée sur une résine Purolite (anionique) pour obtenir le N-méthylcarboxylique acide dextrane carbamate qui est ensuite lyophilisé pendant 18 heures.

5 g de N-méthylcarboxylique acide dextrane carbamate (20 mmol fonctions N-méthylcarboxylique acide) sont solubilisés dans le DMF à 50 g/L puis refroidis à 0°C. 2,22 g (22 mmol) de NMM et 2,38 g (22 mmol) de EtOCOCl sont ensuite ajoutés. Après 10 min de réaction, 0,45 g (1,8 mmol) de N-(2-aminoethyl)dodécanamide est ajouté et le milieu maintenu à 10°C durant 45 minutes. Le milieu est ensuite chauffé à 50°C. A 30°C, une solution aqueuse d'imidazole à 600 g/L et 25 mL d'eau sont ajoutés. Après 1h30 d'agitation à 50°C, la solution obtenue est ultrafiltrée sur membrane PES 10 kD contre NaOH 0,1N, NaCl 0,9% et de l'eau. La concentration de la solution de polymère est déterminée par extrait sec. Une fraction de solution est lyophilisée et analysée par RMN ¹H dans D₂O pour déterminer le taux de fonctions acides converties en amide de N-(2-aminoéthyl)dodécanamide.

D'après l'extrait sec : [Polymère 17] = 17,8 mg/g
D'après la RMN ¹H : le degré de fonctionnalisation des acides par le N-(2-aminoéthyl)dodécanamide par unité saccharidique est de 0,1.

### Exemple 18 : dextranesuccinate de sodium modifié par le N-(2-aminoéthyl)dodécanamide

### Polymère 18

Le N-(2-aminoethyl)dodécanamide est obtenu selon le procédé décrit dans le brevet (Weiner, N. et al., US2387201) à partir du méthyl ester de l'acide dodécanoïque (Sigma) et de l'éthylènediamine (Roth).

Le dextranesuccinate de sodium est obtenu à partir du dextrane 10 selon la méthode décrite dans l'article de Sanchez-Chaves et al. (Sanchez-Chaves, Manuel et al., Polymer 1998, 39 (13), 2751-2757.) Le taux de fonctions acides par unité glycosidique est de 1,41 d'après la RMN ¹H dans D₂O/NaOD.

Par un procédé similaire à celui décrit à l'Exemple 1, un dextranesuccinate de sodium modifié par le N-(2-aminoéthyl)dodécanamide est obtenu.

D'après l'extrait sec : [Polymère 18] = 16,1 mg/g
D'après la RMN ¹H : le degré de fonctionnalisation des acides par le N-(2-aminoéthyl)dodécanamide par unité saccharidique est de 0,05.

### Exemple 19 : dextrane modifié par le N-méthylcarboxylate de sodium carbamate et le N-(2-aminoéthyl)dodécanamide carbamate

### Polymère 19

Le N-(2-aminoéthyl)dodécanamide est obtenu selon le procédé décrit dans le brevet (Weiner, N. et al., US2387201) à partir du méthyl ester de l'acide dodécanoïque (Sigma) et de l'éthylènediamine (Roth).

Le N-(2-isocyanatoéthyl)dodécanamide est obtenu selon le procédé décrit dans la publication (Knöckler, H.-J. et al., Synlett 1997, 925-928) à partir du N-(2-aminoéthyl)dodécanamide.

2,7 g (soit 0,05 mol de fonctions hydroxyles) de dextrane de masse molaire moyenne en poids d'environ 10 kg/mol (Bachem) sont solubilisés dans un mélange DMF/DMSO. Le mélange est porté à 130°C sous agitation et 3.2 g (0,025 mol) d'éthyle isocyanatoacétate puis 2.22 g (0.008 mol) du N-(2-isocyanatoéthyl)dodécanamide sont progressivement introduits. Après 1h de réaction, le milieu est dilué en eau et purifié par diafiltration sur membrane PES de 5 kD contre NaOH 0,1N, NaCl 0,9% et de l'eau. La solution finale est dosée par extrait sec pour déterminer la concentration en polymère. Une fraction de solution est lyophilisée et analysée par RMN ¹H dans D₂O pour déterminer le degré de conversion des fonctions hydroxyles en carbamate de N-méthyl carboxylate de sodium et le degré de fonctionnalisation des fonctions hydroxyles en carbamate de N-(2-aminoéthyl)dodécanamide.

D'après l'extrait sec :[Polymère 19] = 6,5 mg/g
D'après la RMN ¹H : le degré de conversion des fonctions hydroxyles en carbamate de N-méthyl carboxylate de sodium est de 1,1 et le degré de fonctionnalisation des fonctions hydroxyles en carbamate de N-(2-aminoéthyl)dodécanamide est de 0,05.

## Revendications

1. Polysaccharide, **caractérisé en ce qu'**il est choisi parmi les polysaccharides comportant des fonctions carboxyles de formule générale V, lesdits polysaccharides étant monofonctionnalisés : 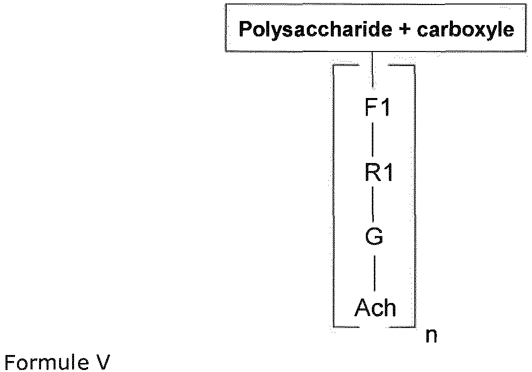
- dans laquelle, n représente le degré de fonctionnalisation des fonctions carboxyles du polysaccharide par un enchaînement F₁-R₁-G-Ach et est compris entre 0,01 et 0,7,
▪ F₁ étant soit une fonction amide, soit une fonction ester, soit une fonction thioester,
▪ G étant soit une fonction ester, soit une fonction amide, soit une fonction thioester,
▪ R₁ étant un radical divalent constitué d'une chaîne comprenant entre 1 et 15 carbones, éventuellement branchée et/ou insaturée, éventuellement comprenant un ou plusieurs hétéroatomes, tels que O, N ou/et S, éventuellement comprenant un ou plusieurs cycles ou hétérocycles saturés, insaturés ou aromatiques et résultant de la réaction d'un précurseur R₁' ayant au moins deux fonctions réactives, identiques ou différentes choisies dans le groupe constitué par les fonctions alcool, amine et thiol.
▪ Ach étant un radical, produit du couplage entre la fonction carboxyle de l'acide hydrophobe et au moins une fonction réactive portée par le précurseur R₁' du radical divalent R₁,
et lorsque la fonction carboxyle du polysaccharide n'est pas fonctionnalisée par F₁-R₁-G-Ach, alors le ou les fonctions carboxyles du polysaccharide sont des carboxylates de cation.

2. Polysaccharide selon la revendication 1, **caractérisé en ce que** le précurseur du groupement R₁, R₁' est choisi parmi les dialcools.

3. Polysaccharide selon la revendication 1, **caractérisé en ce que** le précurseur du groupement R₁, R₁' est choisi parmi les diamines.

4. Polysaccharide selon la revendication 1, **caractérisé en ce que** le précurseur du groupement R₁, R₁' est choisi parmi les alcoolamines.

5. Polysaccharide selon l'une quelconque des revendications précédentes, **caractérisé en ce que** l'acide hydrophobe est choisi parmi les acides gras.

6. Polysaccharide selon l'une quelconque des revendications précédentes, **caractérisé en ce que** le degré de polymérisation m est compris entre 5 et 10000.

7. Procédé de synthèse d'un polysaccharide selon la revendication 1, **caractérisé en ce qu'**il comprend une étape d'obtention d'un intermédiaire aminé Ach-G-R₁-NH₂ ou d'un sel d'ammonium Ach-G-R₁-NH₃+ dont le contre-ion est un anion choisi parmi les halogénures, les sulfates, les sulfonates, les carboxylates, et une étape de greffage de cet intermédiaire aminé sur une fonction carboxyle d'un polysaccharide, R₁, G et Ach répondant aux définitions données dans les revendications précédentes.

8. Composition pharmaceutique comprenant un polysaccharide selon l'une quelconque des revendications 1 à 6 et au moins un principe actif.

## Patentansprüche

1. Polysaccharid, **dadurch gekennzeichnet, dass** es aus Polysacchariden ausgewählt ist, die Carboxylfunktionen tragen mit der allgemeinen Formel V, wobei die Polysaccharide monofunktionalisiert sind: 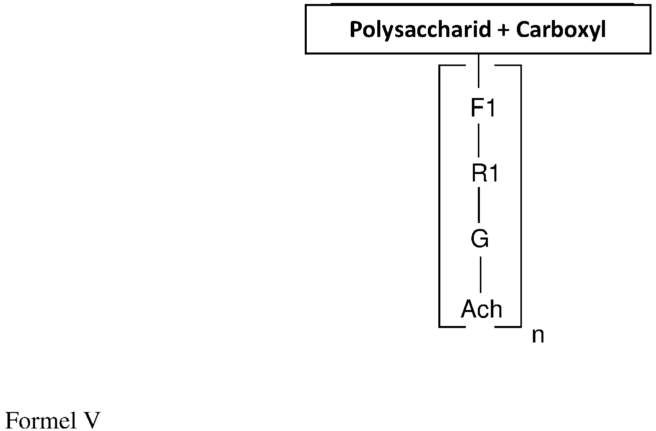 wobei n den Funktionalisierungsgrad der Carboxylfunktionen des Polysaccharids durch eine Sequenz F₁-R₁-G-Ach darstellt und zwischen 0,01 und 0,7 beträgt,
- F₁ eine Amidfunktion, eine Esterfunktion oder eine Thioesterfunktion ist,
- G eine Esterfunktion, eine Amidfunktion oder eine Thioesterfunktion ist,
- R₁ ein divalentes Radikal ist, das aus einer Kette besteht umfassend zwischen 1 und 15 Kohlenstoffatomen, optional verzweigt und/oder ungesättigt, optional umfassend ein oder mehrere Heteroatome wie zum Beispiel O, N und/oder S, optional umfassend einen oder mehrere gesättigte, ungesättigte oder aromatische Heterozyklen, und das Ergebnis der Reaktion eines Vorläufers R₁' mit wenigstens zwei reaktiven Funktionen ist, die identisch oder verschieden und ausgewählt sind aus der Gruppe bestehend aus einer Alkoholfunktion, einer Aminfunktion und einer Thiolfunktion,
- Ach ein Radikal ist, hergestellt durch Kopplung der Carboxylfunktion der hydrophoben Säure mit wenigstens einer reaktiven Funktion, die von dem Vorläufer R₁' des divalenten Radikals R₁ getragen ist,
und wenn die Carboxylfunktion des Polysaccharids nicht durch F₁-R₁-G-Ach funktionalisiert ist, ist/sind die Carboxylfunktion(en) des Polysaccharids kationische Carboxylate.

2. Polysaccharid nach Anspruch 1, **dadurch gekennzeichnet, dass** der Vorläufer der Gruppe R₁ R₁' ausgewählt ist aus Dialkoholen.

3. Polysaccharid nach Anspruch 1, **dadurch gekennzeichnet, dass** der Vorläufer der Gruppe R₁ R₁' ausgewählt ist aus Diaminen.

4. Polysacharid nach Anspruch 1, **dadurch gekennzeichnet, dass** der Vorläufer der Gruppe R₁ R₁' ausgewählt ist aus Alkoholaminen.

5. Polysaccharid nach einem der vorangehenden Ansprüche, **dadurch gekennzeichnet, dass** die hydrophobe Säure ausgewählt ist aus Fettsäuren.

6. Polysaccharid nach einem der vorangehenden Ansprüche, **dadurch gekennzeichnet, dass** der Polymerisationsgrad m zwischen 5 und 10.000 beträgt.

7. Verfahren zur Synthese eines Polysaccharids nach Anspruch 1, **dadurch gekennzeichnet, dass** es umfasst Bereitstellen eines aminierten Zwischenproduktes Ach-G-R₁-NH2 oder eines Ammoniumsalzes Ach-G-R₁-NH₃⁺, dessen Gegenion ein Anion ist, das ausgewählt ist aus Haliden, Sulfaten, Sulfonaten und Carboxylaten, und Aufpropfen des aminierten Zwischenproduktes auf eine Carboxylfunktion eines Polysaccharids, wobei R₁, G und Ach wie in den vorhergehenden Ansprüchen definiert sind.

8. Pharmazeutische Zusammensetzung umfassend ein Polysaccharid nach einem der Ansprüche 1 bis 6 und wenigstens einen Wirkstoff.

## Claims

1. An anionic polysaccharide chosen from polysaccharides comprising carboxyl functions of formula V, said polysaccharides being monofunctionalized: 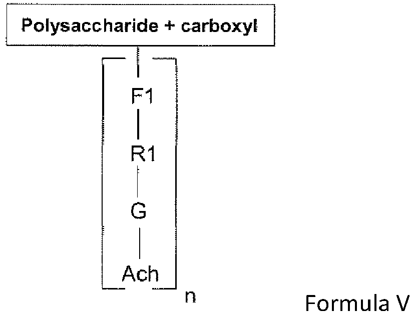
- in which n represents the degree of functionalization of the carboxyl functions of the polysaccharide by an F₁-R₁-G-Ach sequence and is between 0.01 and 0.7,
• F₁ being either an amide function or an ester function or a thioester function,
• G being either an ester function or an amide function or a thioester function,
• R₁ being a divalent radical composed of a chain comprising between 1 and 15 carbon atoms which is optionally branched and/or unsaturated, which optionally comprises one or more heteroatoms, such as O, N and/or S, and which optionally comprises one or more saturated, unsaturated or aromatic rings or heterocycles and resulting from the reaction of a precursor R₁' having at least two identical or different reactive functions chosen from the group consisting of alcohol, amine and thiol functions,
• Ach being a radical which is the product of the coupling between the carboxyl function of the hydrophobic acid and at least one reactive function carried by the precursor R₁' of the divalent radical R₁,
and, when the carboxyl function of the polysaccharide is not functionalized by F₁-R₁-G-Ach, then the carboxyl function or functions of the polysaccharide are cation carboxylates.

2. The polysaccharide as claimed in claim 1, wherein the precursor of the group R₁, R₁', is chosen from dialcohols.

3. The polysaccharide as claimed in in claim 1, wherein the precursor of the group R₁, R₁' is chosen from diamines.

4. The polysaccharide as claimed in in claim 1, wherein the precursor of the group R₁, R₁' is chosen from alcohol amines.

5. The polysaccharide as claimed in any one of the preceding claims, wherein the hydrophobic acid is chosen from fatty acids.

6. The polysaccharide as claimed in any one of the preceding claims, wherein the degree of polymerization m is between 5 and 10 000.

7. A process for the synthesis of a polysaccharide as claimed in claim 1, which comprises a step of producing an aminated intermediate Ach-G-R₁-NH₂ or an ammonium salt Ach-G-R₁-NH₃⁺, the counterion of which is an anion chosen from halides, sulfates, sulfonates or carboxylates, and a step of grafting this aminated intermediate to a carboxyl function of a polysaccharide, R₁, G and Ach corresponding to the definitions given in the preceding claims.

8. A pharmaceutical composition comprising a polysaccharide as claimed in any one of claims 1 to 6 and at least one active principle.
